# EUROPEAN PATENT APPLICATION

(11) **EP 3 805 367 A1**
(43) Date of publication of application: **14.04.2021**
(21) Application number: 20196461.6
(22) Date of filing: 08.12.2006
(51) Int. Cl.: C12N 5/07, C12N 5/00, A61K 39/00, A61P 31/04, A61P 31/12, A61P 35/00, A61P 37/04, C12N 5/0784

(54) **COMPOSITIONS AND METHODS FOR INDUCING THE ACTIVATION OF IMMATURE MONOCYTIC DENDRITIC CELLS**

(30) Priority: 08.12.2005 US 748885 P
(62) Divisional of application: 16170001.8
(71) Applicant: NorthWest Biotherapeutics, Inc., Bethesda, MD 20814 (US)
(72) Inventor: BOYNTON, Alton L., Kingston, Washington 98346-1863 (US); BOSCH, Marnix L., Medina, Washington 98039 (US)
(74) Representative: Forresters IP LLP

(57) **Abstract**

The present invention provides methods for inducing the maturation of immature dendritic cells (DT) and for activating those cells without the use of a dendritic cell maturation agent. The activated DC can be used for inducing an antigen specific T cell response. Methods of the invention can also comprise the addition of a directional maturation agent, such as interferon gamama, to induce a Th-I and/or Th-2 bias in the response obtained. The present invention also provides dendritic cell populationns useful for activating and for inducing antigen specific T cells. Similarly, activated antigen specific T cell populations, and methods of making the same are provided.

## Description

### RELATED APPLICATIONS

This application claims priority to United States Provisional Application No. 60/748,885, filed December 8, 2005, incorporated herein by reference in its entirety.

### BACKGROUND OF THE INVENTION

Antigen presenting cells (APC) are important in eliciting an effective immune response. They not only present antigens to T cells with antigen-specific T cell receptors, but also provide the signals necessary for T cell activation. These signals remain incompletely defined, but involve a variety of cell surface molecules as well as cytokines or growth factors. The factors necessary for the activation and polarization of naive T cells may be different from those required for the re-activation of memory T cells. The ability of APC to both present antigens and deliver signals for T cell activation is commonly referred to as an accessory cell function. Although monocytes and B cells have been shown to be competent APC, their antigen presenting capacities *in vitro* appear to be limited to the re-activation of previously sensitized T cells. Hence, monocytes and B cells are not capable of directly activating functionally naive or unprimed T cell populations. They are also not capable of delivering signals that can polarize an induced immune response, or an immune response as it is induced.

Dendritic cells (DCs) are the professional antigen presenting cells of the immune system that are believed to be capable of activating both naïve and memory T cells. Dendritic cells are increasingly prepared *ex vivo* for use in immunotherapy, particularly the immunotherapy of cancer. The preparation of dendritic cells with optimal immunostimulatory properties requires an understanding and exploitation of the biology of these cells for *ex vivo* culture. Various protocols for the culture of these cells have been described, with various advantages ascribed to each protocol. Recent protocols include the use of serum-free media, and the employment of maturation conditions that impart the desired immunostimulatory properties to the cultured cells.

Activation of dendritic cells initiates the process that converts immature DCs, which are phenotypically similar to skin Langerhans cells, to mature, antigen presenting cells that can migrate to the lymph nodes. This process results in the gradual and progressive loss of the powerful antigen uptake capacity that characterizes the immature dendritic cell, and in the up-regulation of expression of co-stimulatory cell surface molecules and various cytokines. Various stimuli can initiate the maturation of DCs. This process is complex and at least *in vitro* can take up to 48 hours to complete. One other consequence of maturation is a change in the *in vivo* migratory properties of the cells. For example, maturation induces several chemokine receptors, including CCR7, which direct the cells to the T cell regions of draining lymph nodes, where the mature DCs activate T cells against the antigens presented on the DC surface in the context of class I and class II MHC molecules. The terms "activation" and "maturation", and "activated" and "mature" describe the process of inducing and completing the transition from an immature DC (partially characterized by the ability to take up antigen) to a mature DC (partially characterized by the ability to effectively stimulate *de novo* T cell responses). The terms typically are used interchangeably in the art.

Known maturation protocols are based on the *in vivo* environment that DCs are believed to encounter during or after exposure to antigens. The best example of this approach is the use of monocyte conditioned media (MCM) as a cell culture medium. MCM is generated *in vitro* by culturing monocytes and used as a source of maturation factors. (See for example, US 2002/0160430, incorporated herein by reference.) The major components in MCM responsible for maturation are reported to be the (pro)inflammatory cytokines Interleukin 1 beta (IL-1β), Interleukin 6 (IL-6) and tumor necrosis factor alpha (TNFα).

Maturation of DCs therefore can be triggered by a multitude of different factors that act via a host of signal transduction pathways. Consequently, there is no single maturation pathway or outcome, but there exists in fact a universe of mature DC stages, each with their own distinct functional characteristics. Conceptually this makes sense because the various threats to the body that the immune system must respond to are manifold, requiring different attack strategies. As an example, while bacterial infection is best cleared by activated macrophages supplemented with specific antibodies, a viral infection is best attacked through cytotoxic T cells that effectively kill virus-infected cells. The killing of cancer cells typically involves a combination of cytotoxic T cells, natural killer cells and antibodies.

*In vitro* maturation of DCs can therefore be designed to induce the immune system to favor one type of immune response over another, *i.e.,* to polarize the immune response. Directional maturation of DCs describes the notion that the outcome of the maturation process dictates the type of ensuing immune response that results from treatment with the matured DCs. In its simplest form, directional maturation results in a DC population that produces cytokines that direct a T cell response polarized to either a Th1-type or Th2-type response. DCs express up to nine different Toll-like receptors (TLR1 through TLR9), each of which can be used to trigger maturation. Not surprisingly, interaction of bacterial products with TLR2 and TLR4 results in directional maturation of DCs resulting in a polarized response most appropriate to dealing with bacterial infections. Conversely, maturation triggered through TLR7 or TLR9 appears to result more in an anti-viral type response. As an additional example, addition of interferon gamma (IFN-γ) to most maturation protocols results in the production of interleukin 12 by the mature DCs, which dictates a Th1-type response. Conversely, inclusion of prostaglandin E2 has the opposite effect.

Factors that can be used in the directional maturation of activated DCs can therefore include for example, Interleukin 1 beta (IL-1β), Interleukin 6 (IL-6), and tumor necrosis factor alpha (TNFα). Other maturation factors include prostaglandin E2 (PGE2), poly-dIdC, vasointestinal peptide (VIP), bacterial lipopolysaccharide (LPS), as well as mycobacteria or components of mycobacteria, such as specific cell wall constituents. Additional maturation factors include for example, an imidazoquinoline compound, *e.g*., a imidazoquinoline-4-amine compound, such as 4-amino-2-ethoxymethyl-α,α-dimethyl-1H-imidazol[4,5-c]quinolin-1-ethanol (designated R848) or 1-(2-methylpropyl)-1H-imidazo[4,5-c]quinolin-4-amine, and their derivatives (WO 00/47719, incorporated herein by reference in its entirety), a synthetic double stranded polyribonucleotide, *e.g*., poly[I]:poly[C(12)U], and the like, agonists of a Toll-like receptor (TLR), such as TLR3, TLR4, TLR7 and/or TLR9, a sequence of nucleic acids containing unmethylated CpG motifs known to induce the maturation of DC, and the like. Further, a combination of any of the above agents can be used in inducing the maturation of dendritic precursor cells.

Fully mature dendritic cells differ qualitatively and quantitatively from immature DCs. Once fully mature, DCs express higher levels of MHC class I and class II antigens, and higher levels of T cell costimulatory molecules, *i.e.,* CD80 and CD86. These changes increase the capacity of the dendritic cells to activate T cells because they increase antigen density on the cell surface, as well as the magnitude of the T cell activation signal through the counterparts of the costimulatory molecules on the T cells, *e.g*,, CD28 and the like. In addition, mature DCs produce large amounts of cytokines, which stimulate and polarize the T cell response.

Generally methods for *ex vivo* DC generation comprise obtaining a cell population enriched for DC precursor cells from a patient and then differentiating the DC precursor cells *in vitro* into mature DCs prior to introduction back into the patient. Some believe that the DCs must be terminally differentiated, or they will de-differentiate back into monocytes/macrophages and lose much of their immunopotentiating ability. *Ex vivo* maturation of DCs generated from monocytes has been successfully accomplished with the methods and agents listed above.

Typically, to generate immature dendritic cells (DC), one must first purify or enrich the monocytic precursors from other contaminating cell types. This is commonly done through adherence of the monocytic precursors to a plastic (polystyrene) surface, as the monocytes have a greater tendency to stick to plastic than other cells found in, for example, peripheral blood, such as lymphocytes and natural killer (NK) cells. After substantially removing the contaminating cells by vigorous washing, the monocytes are cultured with cytokines that convert the monocytic precursors to either immature DC or directly to mature DC. Methods for differentiating the monocytic precursor cells to immature DC were first described by Sallusto and Lanzavecchia (J. Exp. Med., 179:1109-1118, 1994, incorporated herein by reference), who used the cytokines GM-CSF and IL-4 to induce the differentiation of the monocytes to immature DC. While this combination of cytokines is most typically used, various other combinations have been described to accomplish the same goals, such as replacing IL-4 with IL-13 or IL-15. The end result of this process is a "veiled" cell, which expresses T cell costimulatory molecules, as well as high levels of molecules of the major histocompatibility complex (MHC), but does not express the dendritic cell maturation marker CD83. These cells are similar to Langerhans cells in the skin, and their prime physiological function is to capture invading microorganisms.

Variations on this method include different methods of purifying monocytes, including, for example, tangential flow filtration (TFF), or by binding antibodies attached to beads to surface molecules on the monocytes. The beads with the bound cells are then concentrated in a column, or on a magnetic surface, such that contaminating cells can be washed away, after which the monocytes are eluted off the beads. In yet another method to obtain dendritic cells precursors, cells expressing the stem cell marker CD34, either from blood (U.S. Patent No. 5,994,126, incorporated herein by reference) or from the bone marrow are purified. These cells can be cultured with the essential cytokine GM-CSF to differentiate into immature DC. These DC apparently have very similar characteristics and functional properties as immature DC generated from monocytes.

Immature DC have a high capacity for taking up and processing antigen, but have a limited ability to initiate immune responses. The ability to initiate an immune response is acquired by maturation of the immature DC. This maturation is also referred to as activating, or activation of, the DC. The maturation process is initiated through contact with maturation-inducing cytokines, bacterial products or viral components, and the like, as set forth above.

While these methods are capable of producing mature DC, there are disadvantages to using recombinant molecules and cellular supernatants for DC maturation. These include inconsistent quality and yield from lot to lot of these reagents and the introduction of large amounts of exogenous proteins that may compete with the antigen of interest for transport into the monocytic dendritic cell precursor for processing. The exogenous proteins may also be toxic or result in autoimmunity if administered to patients. Such reagents can also be expensive to produce, making the cost of immunotherapy prohibitively expensive.

### BRIEF SUMMARY

The presently described methods and compositions provide for the induction of activation of immature dendritic cells (DC), and for the priming of those cells for an antigen-specific immune response. In one aspect, a method is provided for producing a cell population enriched for activated dendritic cells by contacting immature dendritic cells with a tissue culture substrate and a culture medium having a dendritic cell differentiation inducing agent under culture conditions suitable for adhesion of the dendritic cells with the tissue culture substrate and for *in vitro* culture of the immature dendritic cells for a time period sufficient to form a cell population enriched for activated dendritic cells. In the described methods it is not necessary to add a dendritic cell maturation agent to induce the activation of the dendritic cell population. The immature dendritic cells can be contacted with a predetermined antigen either prior to, during, or after the contacting of the immature dendritic cells with the tissue culture surface. The predetermined antigen can be, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*e.g*., a synthetic peptide antigen), or an isolated antigen. Further, the antigen can be either a soluble antigen or a particulate antigen. In a particular embodiment the antigen can be a cell lysate or a membrane preparation derived from a patient tumor or a tumor cell line.

In certain embodiments, the method can optionally further include obtaining a cell population enriched for monocytic dendritic cell precursors; and culturing the precursors in the presence of an agent that can induce differentiation of the monocytic dendritic cell precursor cells to form a population of immature dendritic cells. Suitable dendritic cell differentiation agents include, for example, GM-CSF, Interleukin 4, a combination of GM-CSF and Interleukin 4, or GM-CSF and Interleukin 13 or Interleukin 15. The monocytic dendritic cell precursors can be obtained as cells isolated from a human subject.

In another aspect, a method for producing a cell population enriched for activated dendritic cells is provided. The method generally includes providing a cell population enriched for immature dendritic cells; and contacting the immature dendritic cells with a tissue culture substrate and a culture medium having a dendritic cell differentiation inducing agent under culture conditions suitable for adhesion of the dendritic cells with the tissue culture substrate and for *in vitro* culture of the immature dendritic cells. The cells are cultured for a time period sufficient to form a cell population enriched for activated mature dendritic cells. The resulting activated mature dendritic cell population can, when administered to a mammal, produce an antigen specific immune response in that mammal. The immature dendritic cell population can be contacted with a predetermined antigen prior to, during, or after contacting with the tissue culture substrate under conditions suitable for adhesion. The predetermined antigen can be, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*i.e.*, a synthetic peptide), or an isolated antigen. The antigen can be either a soluble antigen or a particulate antigen. In a particular embodiment the antigen is a cell lysate or a membrane preparation derived from a patient tumor or a tumor cell line. An agent that can direct the maturation of the DCs to bias the response to a Th1 and/or a Th2 response, *e.g*., interferon gamma (IFNγ), can also be added.

In certain embodiments, the method can optionally further include obtaining monocytic dendritic cell precursors; and culturing the precursors *in vitro* in the presence of a differentiation agent to form the immature dendritic cells. Suitable differentiation agents include, for example, GM-CSF, Interleukin 4, Interleukin 13, Interleukin 15, or combinations thereof. The monocytic dendritic cell precursors can be isolated from a human subject in need of treatment or from a histocompatibility matched individual.

In still another aspect, compositions for activating T cells are provided. The compositions can include a dendritic cell population activated and matured by contact with a tissue culture substrate and a dendritic cell differentiation inducing agent under suitable conditions for adhesion of the dendritic cells to the tissue culture substrate and for activation; and a predetermined antigen. The dendritic cell population can produce an antigen specific immune response similar to that induced by a mature dendritic cell population produced by a prior method. The dendritic cell population is produced by isolating immature dendritic cells from a prior culture media and added cytokines and contacting the isolated immature dendritic cells with a tissue culture substrate under conditions suitable for adhesion of the DCs to the tissue culture substrate without the addition of a dendritic cell maturation agent. The dendritic cells upon contact with the tissue culture substrate under conditions suitable for dendritic cell adhesion to the tissue culture substrate are triggered to proceed through activation and maturation into active mature dendritic cells. Predetermined soluble or particulate antigen added either simultaneously with the immature dendritic cells to the tissue culture substrate or added during the maturation process, *i.e.,* after activation but before completion of maturation, can be taken in and processed by the dendritic cells and presented in the context of the appropriate cell surface receptors to be available upon contact with T cells to induce an antigen specific immune response.

In another aspect, an isolated, activated dendritic cell population is provided. The cell population includes and is enriched for mature activated monocytic dendritic cells previously contacted with a tissue culture substrate and a dendritic cell differentiation inducing agent under conditions conducive for adherence of the dendritic cells with the tissue culture substrate. The resulting activated dendritic cells can uptake and process antigen and upon continued culture *in vitro,* can acquire the cell surface phenotype of a mature dendritic cell. The cell population can optionally include a predetermined antigen and/or isolated T cells, such as naïve T cells. The T cells can optionally be present in a preparation of isolated lymphocytes.

A method for producing activated T cells is also provided. The method generally includes providing a cell population enriched for isolated immature dendritic cells; contacting the immature dendritic cells with a predetermined antigen and contacting the immature dendritic cells with a tissue culture substrate and a dendritic cell differentiation inducing agent under conditions conducive to adhesion of the immature dendritic cells to the tissue culture substrate. The cells are cultured for a time period sufficient to induce activation of the immature dendritic cells to form activated dendritic cells. The activated dendritic cells can be contacted with naïve T cells to form activated antigen specific T cells. Suitable antigens can include, for example, a tumor specific antigen, a tumor associated antigen, a viral antigen, a bacterial antigen, tumor cells, bacterial cells, recombinant cells expressing an antigen, a cell lysate (including tumor cell lysate), a membrane preparation, a recombinantly produced antigen, a peptide antigen (*e.g*., a synthetic peptide antigen), or an isolated antigen. The predetermined antigen can be either a soluble antigen or a particulate antigen. An agent that can direct the maturation of the DCs to bias the response to a Th1 and/or a Th2 response, *e.g*., interferon gamma, can also be added.

The cell population enriched for immature dendritic cells can be contacted simultaneously with the predetermined antigen, and the tissue culture substrate and dendritic cell differentiation inducing agent, or the cells can be contacted with the predetermined antigen prior to, during or simultaneously with, or after contacting with the tissue culture substrate and the dendritic cell differentiation inducing agent. In certain embodiments, the method can further include obtaining a cell population enriched for monocytic dendritic cell precursors; and culturing the precursors *in vitro* in the presence of the dendritic cell differentiation inducing agent to induce the formation of the immature dendritic cells. Suitable differentiation inducing agents include, for example, GM-CSF, Interleukin 4, Interleukin 13 or Interleukin 15, or combinations thereof. The monocytic dendritic cell precursors can optionally be obtained from a human subject. In a particular embodiment, the monocytic dendritic precursor cells, immature dendritic cells, and/or T cells are autologous to each other.

The activated antigen specific T cells can be administered to an animal, particularly a mammal, in need of stimulation of an antigen specific immune response. Suitable antigens include, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*e.g*., a synthetic peptide antigen), or an isolated antigen. In a particular embodiment the cell lysate and/or membrane preparation is derived from a patient tumor or a tumor cell line. The immature dendritic cells can optionally be simultaneously contacted with the predetermined antigen, and a tissue culture substrate, a dendritic cell differentiation inducing agent, or the immature dendritic cells can be contacted with the predetermined antigen prior to contacting with the new, unused, clean tissue culture substrate.

In certain embodiments, the method can further include isolating monocytic dendritic cell precursors from the animal; and culturing the precursors *in vitro* in the presence of a differentiating agent to form the immature dendritic cells. The differentiating agent can be, for example, GM-CSF, Interleukin 4, Interleukin 13, Interleukin 15, or combinations thereof.

The monocytic dendritic cell precursor immature dendritic cell population, and/or T cells can be autologous to the animal, or allogenic to the animal. Alternatively, the monocytic dendritic cell precursors, immature dendritic cells and/or T cells can have the same MHC haplotype as the animal, or share an MHC marker. In certain embodiments, the animal can be human, or can be a non-human animal.

### DETAILED DESCRIPTION

The present invention provides methods for inducing or triggering activation and the maturation of immature dendritic cell (DC) populations and for priming those cell populations for an antigen-specific immune response. The immature dendritic cell populations can be obtained from preparation media, including for example, isolation media, culture media, and the like, and the cell population enriched for immature monocytic dendritic cells are contacted with a tissue culture substrate and a dendritic cell differentiation inducing agent under conditions suitable for adhesion of the DCs to the tissue culture substrate without the addition of a dendritic cell maturation agent. The immature dendritic cells can be contacted with a predetermined antigen under suitable *in vitro* cell culture conditions for uptake processing and presentation by the DCs. Contact with the antigen of interest can be either during, prior to, or subsequent to the initiation of activation. Alternatively, immature monocytic dendritic cells, already exposed to antigen (*e.g., in vivo*)*,* can be obtained and contacted with a tissue culture substrate under similarly suitable cell culture conditions. The resulting activated mature dendritic cells present the antigen of interest and are primed to activate T cells towards an antigen specific response. Direction of the response, *i.e.,* bias of the response ion favor of a Th-1 or Th-2 response can be influenced by the addition of a directional maturation agent, such as interferon gamma (IFNγ) and the like.

In another aspect, a cell population enriched for monocytic dendritic cell precursors can be obtained from a subject or from a donor. The cell population can be contacted with a dendritic cell differentiation inducing agent such as, for example, one or more cytokines (*e.g*. but not limited to, GM-CFS, and combinations of GM-CSF and IL-4, GM-CSF and IL-13, GM-CSF and IL-15, and the like) to obtain immature dendritic cells. The immature dendritic cells can then be contacted with a predetermined antigen, either in combination with a tissue culture substrate and a dendritic cell differentiation inducing agent, or a cytokine or other directional maturation agent, to activate and/or partially mature the dendritic cells. The mature dendritic cells can be used directly to induce an antigen specific immune response in a subject or the cells can be used for inducing an antigen specific immune response in T cells. In certain embodiments, MHC Class-I antigen processing is stimulated, which is useful to elicit a CTL response against cells displaying the predetermined antigen. The direction of the response induced can be influenced by the addition of a directional maturation agent, such as interferon gamma. As used herein a directional maturation agent is an agent that can affect the final state of the mature DCs but does not induce DC maturation when used alone. For example, a directional maturation agent can polarize the maturation of a DC population to favor a Th1 versus a Th-2 response, or vice versa.

Dendritic cells are a diverse population of antigen presenting cells found in a variety of lymphoid and non-lymphoid tissues. (*See* Liu, Cell 106:259-262 (2001); Steinman, Ann. Rev. Immunol. 9:271-296 (1991)). Dendritic cells include lymphoid dendritic cells of the spleen, Langerhans cells of the epidermis, and veiled cells in the blood circulation. Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD11c, CD80, CD83, CD86, and are HLA-DR⁺, but are typically, but not always, CD14⁻.

In contrast, monocytic dendritic cell precursors (typically monocytes) are usually CD14⁺ and express no or low levels of HLA-DR, CD83, and CD86. Monocytic dendritic cell precursors can be obtained from any tissue where they reside, particularly lymphoid tissues such as the spleen, bone marrow, lymph nodes and thymus. Monocytic dendritic cell precursors also can be obtained from the circulatory system. Peripheral blood is a readily accessible source of monocytic dendritic cell precursors. Umbilical cord blood is another source of monocytic dendritic cell precursors. Monocytic dendritic cell precursors can be obtained from a variety of organisms in which an immune response can be elicited. Such organisms include animals, for example, including humans, and non-human animals, such as, primates, other mammals (including, but not limited to, dogs, cats, mice, and rats), birds (including chickens), as well as transgenic species thereof.

In certain embodiments, the cell population enriched for monocytic dendritic cell precursors and/or immature dendritic cells can be obtained from a healthy subject or, in the alternative, from a subject in need of immunostimulation, such as, for example, a cancer patient, (*e.g*., cancer of the brain, breast, ovary, lung, prostate, colon, and the like) or another subject for whom cellular immunostimulation can be beneficial or desired (*i.e.,* a subject having a bacterial or viral infection, and the like). Dendritic cell precursors and/or immature dendritic cells also can be obtained from an HLA-matched healthy individual for administration to an HLA-matched subject in need of immunostimulation.

### Dendritic Cell Precursors and Immature Dendritic Cells

Methods for isolating cell populations enriched for dendritic cell precursors and immature dendritic cells from various sources, including blood and bone marrow, are known in the art. For example, cell populations enriched for dendritic cell precursors and immature dendritic cells can be obtained by collecting heparinized blood, by apheresis or leukapheresis, by preparation of buffy coats, rosetting, centrifugation, density gradient centrifugation (*e.g*., using FICOLL (such as FICOLL-PAQUE®), PERCOLL® (colloidal silica particles (15-30 mm diameter) coated with non-dialyzable polyvinylpyrrolidone (PVP)), sucrose, and the like), differential lysis of cells, filtration, and the like. In certain embodiments, a leukocyte population can be prepared, such as, for example, by collecting blood from a subject, defribrinating to remove the platelets and lysing the red blood cells. Dendritic cell precursors and immature dendritic cells can optionally be enriched for monocytic dendritic cell precursors by, for example, centrifugation through a density gradient material, such as for example a PERCOLL® gradient.

All populations enriched for dendritic cell precursors and immature dendritic cells optionally can be prepared in, for example, a closed, aseptic system. As used herein, the terms "closed, aseptic system" or "closed system" refer to a system in which exposure to non-sterilized, ambient, or circulating air or other non-sterile conditions is minimized or eliminated. Closed systems for obtaining dendritic cell precursors and immature dendritic cells generally exclude density gradient centrifugation in open top tubes, open air transfer of cells, culture of cells in tissue culture plates or unsealed flasks, and the like. In a typical embodiment, the closed system allows aseptic transfer of the dendritic cell precursors and immature dendritic cells from an initial collection vessel to a sealable tissue culture vessel without exposure to non-sterile air.

In certain embodiments, monocytic dendritic cell precursors are obtained by adherence of monocytes to a monocyte-binding substrate, as disclosed in WO 03/010292, the disclosure of which is incorporated by reference herein. For example, a population of leukocytes (*e.g*., isolated by leukapheresis) can be contacted with a monocytic dendritic cell precursor adhering substrate. When the population of leukocytes is contacted with the substrate, the monocytic dendritic cell precursors in the leukocyte population preferentially adhere to the substrate. Other leukocytes (including other potential dendritic cell precursors) exhibit reduced binding affinity to the substrate, thereby allowing the monocytic dendritic cell precursors to be preferentially enriched on the surface of the substrate.

Suitable substrates include, for example, those having a large surface area to volume ratio. Such substrates can be, for example, a particulate or fibrous substrate. Suitable particulate substrates include, for example, glass particles, plastic particles, glass-coated plastic particles, glass-coated polystyrene particles, and other beads suitable for protein absorption. Suitable fibrous substrates include microcapillary tubes and microvillous membrane. The particulate or fibrous substrate usually allows the adhered monocytic dendritic cell precursors to be eluted without substantially reducing the viability of the adhered cells. A particulate or fibrous substrate can be substantially non-porous to facilitate elution of monocytic dendritic cell precursors or dendritic cells from the substrate. A "substantially non-porous" substrate is a substrate in which at least a majority of pores present in the substrate are smaller than the cells to minimize entrapping cells in the substrate.

Adherence of the monocytic dendritic cell precursors to the substrate can optionally be enhanced by addition of a binding media. Suitable binding media include monocytic dendritic cell precursor culture media (*e.g*., AIM-V®, RPMI 1640, DMEM, X-VIVO 15®, and the like) supplemented, individually or in any combination, with for example, cytokines (*e.g*., Granulocyte/Macrophage Colony Stimulating Factor (GM-CSF), Interleukin 4 (IL-4),or Interleukin 13 (IL-13)), blood plasma, serum (*e.g.,* human serum, such as autologous or allogenic sera), purified proteins, such as serum albumin, divalent cations (*e.g.*, calcium and/or magnesium ions) and other molecules that aid in the specific adherence of monocytic dendritic cell precursors to the substrate, or that prevent adherence of non-monocytic dendritic cell precursors to the substrate. In certain embodiments, the blood plasma or serum can be heated-inactivated. The heat-inactivated plasma can be autologous or heterologous to the leukocytes.

Following adherence of monocytic dendritic cell precursors to the substrate, the non-adhering leukocytes are separated from the monocytic dendritic cell precursor/substrate complexes. Any suitable means can be used to separate the non-adhering cells from the complexes. For example, the mixture of the non-adhering leukocytes and the complexes can be allowed to settle, and the non-adhering leukocytes and media decanted or drained. Alternatively, the mixture can be centrifuged, and the supernatant containing the non-adhering leukocytes decanted or drained from the pelleted complexes.

In another method, all populations enriched for monocytic dendritic cell precursors can be obtained from a cell population enriched in leukocytes prepared by the use of a tangential flow filtration device. A tangential flow filtration device useful for obtaining the cell population enriched in monocytic dendritic cell precursors can comprise a remover unit having a cross-flow chamber, a filtrate chamber and a filter disposed there between. (*See* WO 2004-000444, incorporated herein by reference in its entirety.) The filter is in fluid communication on one side, the retentate surface, with the cross-flow chamber, and on the other side, the filtrate surface, with the filtrate chamber. The cross-flow chamber has an inlet adapted to introduce a sample of blood constituents comprising leukocytes into the cross-flow chamber and parallel to the retentate surface of the filter. An outlet is also provided in the cross-flow chamber centrally disposed in a portion of the chamber opposite the retentate surface of the filter. The filter suitable for use in the tangential flow filtration device typically has an average pore size ranging from about 1 to about 10 microns. The filter can have an average pore size of about 3 to about 7 microns. A means for providing a predetermined input rate of the sample into the inlet of the cross-flow chamber and a means for controlling a filtration rate of filtrate through the filter and into the filtrate chamber can also be included. The filtration rate controlling means limits the rate of filtration to less than the unopposed filtration rate for the filter. The sample comprising blood constituents can be provided by a source device such as a leukapheresis device or a container comprising a sample collected from a leukapheresis device.

The dendritic cell precursors can be cultured *in vitro* or *ex vivo* for differentiation, maturation and/or expansion. (As used herein, isolated immature dendritic cells, dendritic cell precursors, T cells, and other cells, refers to cells that, by human hand, exists apart from their native environment, and are therefore not a product of nature. Isolated cells can exist in purified form, in semi-purified form (for example, an enriched cell population), or in a non-native environment. Briefly, *ex vivo* differentiation typically involves culturing dendritic cell precursors, or populations of cells having dendritic cell precursors, in the presence of one or more dendritic cell differentiation agents. Suitable differentiation inducing agents can be, for example, but not limited to, cellular growth factors (*e.g*., cytokines such as (GM-CSF), Interleukin 4 (IL-4), Interleukin 13 (IL-13), Interleukin 15 (IL-15), and/or combinations thereof). In certain embodiments, the monocytic dendritic cells precursors are induced to differentiate from monocyte-derived immature dendritic cells.

The dendritic cell precursors can be cultured and induced to differentiate under suitable culture conditions. Suitable tissue culture media include, for example, AIM-V®, RPMI 1640, DMEM, X-VIVO 15®, and the like. The tissue culture media can be supplemented with serum, amino acids, vitamins, cytokines, such as GM-CSF and/or IL-4, IL-13, or IL-15, divalent cations, and the like, to promote differentiation of the cells into immature dendritic cells. In certain embodiments, the dendritic cell precursors can be cultured *in vitro* in a serum-free media. Such culture conditions can optionally exclude any animal-derived products. A common cytokine combination in a typical dendritic cell culture medium comprises about 500 units/ml each of GM-CSF and IL-4. Dendritic cell precursors, when differentiated to form immature dendritic cells, are phenotypically similar to skin Langerhans cells. Immature dendritic cells typically are CD14⁻ and CD11c⁺, express low levels of CD86 and CD83, and are able to capture soluble antigens via specialized endocytosis.

Typically, in prior methods the immature dendritic cells are matured *in vitro* or *ex vivo* to form mature dendritic cells prior to administration to a patient or prior to contact with T cells. In these methods, a dendritic cell maturation agent is added to the *in vitro* culture comprising the immature dendritic cells either prior to or with a predetermined antigen. Upon maturation, the DCs gradually and progressively lose the ability to take up antigen and they typically display up-regulated expression of costimulatory cell surface molecules and various cytokines. Specifically, mature DC express higher levels of MHC class I and II antigens than immature dendritic cells, and mature dendritic cells are generally identified as being CD80⁺, CD83⁺, CD86⁺, and CD14⁻. Greater MHC expression leads to an increase in antigen density on the DC surface, while up regulation of costimulatory molecules CD80 and CD86 strengthens the T cell activation signal through the counterparts of the costimulatory molecules, such as CD28, on the T cells.

Unlike prior methods, activation of the immature dendritic cells in the method of the present invention is initiated or triggered by contacting isolated immature dendritic cells with a tissue culture substrate and a dendritic cell differentiation inducing agent under conditions suitable for adherence of the dendritic cell to the tissue culture surface. In a typical method of the present invention, activation is achieved without the addition of a maturation agent. The immature dendritic cells are removed from a prior culture substrate or purification media and isolated from the prior culture media. The isolated immature dendritic cells are then counted and frozen for later use of combined with a fresh culture media without added dendritic cell maturation factors for the remainder of the process. In one alternative method, a directional maturation agent can be added during activation to bias the dendritic cells so that they can polarize a T cell response to a Th-1 or a Th-2 response. As an example, but not as a limitation to the agents that can be used, interferon gamma can be added to bias the T cell response toward a Th-1 response. Interferon gamma added to monocytic dendritic cell precursors or immature DCs by itself does not induce DC differentiation and/or maturation.

Tissue culture substrates useful in the methods of the present invention can comprise a tissue culture well, flask, bottle, bag or any matrix used in a bioreactor, such as fiber, beads, plates, and the like. Typically, tissue culture substrates comprise plastics, such as polystyrene, Teflon® (polytetrafluoroethylene, PTFE), and the like. Those tissue culture substrates most often used for the *ex vivo* culture of dendritic cells for immunotherapy comprise tissue culture flasks, -bags, or cell fractions which are comprised of multiple stacked layers of plastic, and the like.

The isolated immature dendritic cells can be cultured and matured in suitable maturation culture conditions. Suitable tissue culture media include AIM-V®, RPMI 1640, DMEM, X-VIVO 15®, and the like. The tissue culture media can be supplemented with amino acids, vitamins, cytokines, human serum, for example, about 1% to about 10% human AB serum, divalent cations, and the like, to promote maturation of the cells.

Maturation or activation of dendritic cells can be monitored by methods known in the art. Cell surface markers can be detected in assays familiar to the art, such as flow cytometry, immunohistochemistry, and the like. The cells can also be monitored for cytokine production (*e.g*., by ELISA, FACS, or other immune assay). In a DC population activated according to the present invention, cells can also be assayed for the appearance of typical cell surface markers CD83, CD86 and HLA-DR. Some of these antigens like CD83 are expressed only on mature DCs, whereas the expression of others is significantly up-regulated upon maturation. Mature DCs also lose the ability to uptake antigen by pinocytosis, which can be analyzed by uptake assays familiar to one of ordinary skill in the art. Dendritic cell precursors, immature dendritic cells, and mature dendritic cells, either primed or unprimed, with antigens can be cryopreserved for use at a later date. Methods for cryopreservation are well-known in the art. *See,* for example, U.S. Patent 5,788,963 incorporated herein by reference in its entirety.

### Antigens

The mature, activated dendritic cells according to the present invention can present antigen to T cells. Mature, activated dendritic cells can be formed by contacting immature dendritic cells with a predetermined antigen either during or subsequent to activation. Alternatively, immature dendritic cells that have already been contacted with antigen (*e.g*., *in vivo* prior to isolation) can be contacted with the tissue culture substrate and a dendritic cell differentiation inducing agent to produce mature dendritic cells activated for inducing a cytotoxic T cell response.

Suitable predetermined antigens can include any antigen for which T-cell activation is desired. Such antigens can include, for example, bacterial antigens, tumor specific or tumor associated antigens *(e.g.,* whole cells, tumor cell lysate, *e.g.,* lysates from glioblastoma, prostate, or ovarian, breast, colon, brain, melanoma, or lung tumor cells, and the like), isolated antigens from tumors, fusion proteins, liposomes, and the like, viral antigens, and any other antigen or fragment of an antigen, *e.g*., a peptide or polypeptide antigen. In certain embodiments, the antigen can be, for example, but not limited to, prostate specific membrane antigen (PSMA), prostatic acid phosphatase (PAP), or prostate specific antigen (PSA). (*See, e.g.,* Pepsidero et al, Cancer Res. 40:2428-32 (1980); McCormack et al., Urology 45:729-44 (1995).) The antigen can also be a bacterial cell, bacterial lysate, membrane fragment from a cellular lysate, or any other source known in the art. The antigen can be expressed or produced recombinantly, or even chemically synthesized. The recombinant antigen can also be expressed on the surface of a host cell (*e.g*., bacteria, yeast, insect, vertebrate or mammalian cells), can be present in a lysate, or can be purified from the lysate. The antigen can be either a soluble antigen or a particulate antigen.

Antigen can also be present in a sample from a subject. For example, a tissue sample from a hyperproliferative or other condition in a subject can be used as a source of antigen. Such a sample can be obtained, for example, by biopsy or by surgical resection. Such an antigen can be used, for example, as a lysate or as an isolated preparation. Alternatively, a membrane preparation of cells of a subject (*e.g.,* a cancer patient), or an established cell line also can be used as an antigen or source of antigen.

In an exemplary embodiment, a glioblastoma tumor cell lysate recovered from surgical specimens can be used as a source of antigen. For example, a sample of a cancer patient's own tumor, obtained at biopsy or at surgical resection, can be used directly to present antigen to dendritic cells or to provide a cell lysate for antigen presentation. Alternatively, a membrane preparation of tumor cells of a cancer patient can be used. The tumor cell can be glioblastoma, prostate, lung, ovarian, breast, colon, brain, melanoma, or any other type of tumor cell. Lysates and membrane preparations can be prepared from isolated tumor cells by methods well known in the art.

In one embodiment, the monocytic dendritic cell precursors can be isolated on a substrate, eluted from the substrate, and transferred to a bioreactor, or other closed system, such as a tissue culture bag. Suitable tissue culture bags include, for example, STERICELL culture containers (Nexell Therapeutics Inc.) or TEFLON culture bags (American Fluoroseal Corp.), and the like. The closed system can have any suitable size or volume, as will be appreciated by the skilled artisan. Suitable volumes include, for example, from about 0.01 liters to about 5 liters, or about 0.01 liters to about 0.05 liters, although greater and lesser volumes are possible and within the scope of the present invention.

The monocytic dendritic cell precursors can also be cultured on the substrate. For example, the monocytic dendritic cell precursors on the substrate can be cultured in a bioreactor (including a fermenter) or tissue culture vessels, such as tissue culture flasks, bags, or plates. The tissue culture flasks, -bags or plates can have any suitable size or volume, as will be appreciated by the skilled artisan. A bioreactor typically has a volume of from about 0.01 to about 5 liters, or about 0.01 to about 0.05 liters, although greater and lesser volumes are possible and within the scope of the present invention. Typically, the bioreactor used for culture of monocytic dendritic cell precursors will have a volume of about 0.01 to about 0.1 liters. The bioreactor can be inoculated with any suitable amount of monocytic dendritic cell precursors, such as, for example, from about 10⁵ cells to about 5 x 10⁶ cells per milliliter of substrate. The monocytic dendritic cell precursors on the substrate can also be cultured in a closed, aseptic system.

The monocytic dendritic cell precursors are cultured and differentiated to obtain immature dendritic cells. Suitable tissue culture media include AIM-V, RPMI 1640, DMEM, X-VIVO15 and the like. The tissue culture medium can be supplemented with amino acids, vitamins, cytokines, such as granulocyte/macrophage colony stimulating factor (GM-CSF) and/or interleukin 4 (IL-4), interleukin 7 (IL-7) or interleukin 13 (IL-13), divalent cations, and the like, to promote differentiation of the monocytic dendritic cell precursors to immature dendritic cells. A typical cytokine combination is about 500 units/ml of each of GM-CSF and IL-4. Typically, if the monocytic dendritic cell precursors are cultured on the substrate the number of mature dendritic cells recovered as the cells unadhere from the substrate surface are primarily mature dendritic cells. The monocytic dendritic cell precursors can be cultured for any suitable time.

In certain embodiments, suitable culture times for the differentiation of precursors to immature dendritic cells can be about 4 to about 7 days. The differentiation of immature dendritic cells from the precursors can be monitored by methods known to those skilled in the art, such as by monitoring the presence or absence of cell surface markers, such as, CD14, CD11c, CD83, CD86, HLA-DR, with labeled monoclonal antibody. The phenotype of the dendritic cells can also be determined by analysis of patterns of gene expression by methods well known in the art. A typical cell surface phenotype for immature dendritic cells would be CD14⁻, CD11c⁺, CD83⁻, CD86⁻, and HLA-DR⁺. Immature dendritic cells can also be cultured in appropriate tissue culture medium to expand the cell population and/or maintain the immature dendritic cells in a state for further differentiation or antigen uptake, processing and presentation. For example, immature dendritic cells can be maintained and/or expanded in the presence of GM-CSF and IL-4.

Immature dendritic cells can be preferred for some applications because they retain the ability to process new antigen. (See, *e.g.,* Koch et al., J. Immunol. 155: 93-100 (1995)). In contrast, mature dendritic cells (*e.g.,* CD14⁻, CD11c⁺, CD83⁺, CD86⁺, HLA-DR⁺), those that have been exposed to and process antigen and to suitable maturation conditions, have typically lost the ability to efficiently process new antigens. Mature dendritic cells can be contacted with peptides that are capable of binding to MHC class I and/or MHC class II molecules for presentation on the cell surface.

During culture, immature dendritic cells can optionally be exposed to a predetermined antigen. Suitable predetermined antigens can include as above any antigen for which T-cell activation is desired. In one embodiment, immature dendritic cells are cultured in the presence of a tumor cell lysate, such as a glioblastoma, prostate, lung, ovary, breast, colon, brain, melanoma, tumor cell lysate, and the like, for cancer immunotherapy and/or tumor growth inhibition. Other antigens can include, for example, bacterial and viral antigens, tumor cells, purified tumor cell membrane, tumor specific or tumor associated antigens (*e.g*., isolated antigens from tumors, fusion proteins, liposomes, and the like), bacterial cells, bacterial antigen, viral particles, viral antigens, and any other antigen. In addition, an antigen can be expressed on the surface of a transformed or transfected host cell expressing the antigen, or a purified membrane or a cell lysate of a transfected or transformed cell expressing the antigen of interest.

Following contacting with antigen, such as tumor cell lysate, the cells can be cultured for any suitable time to allow antigen uptake and processing, to expand the population of antigen-specific dendritic cells, and the like. Immature dendritic cells can also be matured into activated dendritic cells that present antigen in the context of MHC class I or MHC class II molecules. Such maturation can be performed, for example, by culture on a tissue culture substrate and with a dendritic cell differentiation inducing agent under conditions conducive to dendritic cell adhesion to the tissue culture substrate in the absence of other known maturation agents. Typically, the dendritic cell differentiation inducing agent is GM-CSF and IL-4, IL-13 or IL-15, and the like.

According to another aspect, dendritic cells can be exposed to a predetermined antigen and a directional maturation agent. A directional maturation agent does not, when used alone, induce the differentiation of monocytic dendritic precursor cells or induce the maturation of immature dendritic cells, but typically when combined with an activation process direct the maturation of the DCs toward cells that can induce a Th-1 or Th-2 response. Interferon gamma is an example of an agent that can induce the bias of the induced T cell response toward a Th-1 response.

In another embodiment of the present invention, immature dendritic cells exposed to a predetermined antigen can be used to activate T cells *in vitro* against an antigen. The dendritic cells can be used immediately after exposure to antigen to stimulate T cells. Alternatively, dendritic cells can be maintained in the presence of a combination of cytokine (*e.g*., GM-CSF and IL-4) prior to exposure to antigen and T cells, or the dendritic cells can be cryopreserved by methods well known in the art for use at a later time. In a specific embodiment, human dendritic cells are used to stimulate human T cells.

T cells or a subset of T cells can be obtained from various lymphoid tissues for use as responder cells. Such tissues include but are not limited to the spleen, lymph nodes, and peripheral blood. The isolated or purified T cells can be co-cultured with dendritic cells exposed to the predetermined antigen as a mixed T cell population or as a purified T cell subset.

For example, purified CD8⁺ T cells can be co-cultured with antigen-exposed dendritic cells to elicit an antigen-specific CTL response. In addition, early elimination of CD4⁺ T cells can prevent the overgrowth of CD4⁺ cells in a mixed culture of both CD8⁺ and CD4⁺ T cells. T cell purification can be achieved by positive or negative selection including, but not limited to, the use of antibodies directed to CD2, CD3, CD4, CD6, and/or CD8.

Alternatively, mixed populations of CD4⁺ and CD8⁺ T cells can be co-cultured with dendritic cells to elicit a response specific to an antigen encompassing both a cytotoxic and T helper immune response. In certain embodiments, activated CD8⁺ or CD4⁺ T cells can be generated according to the method of the present invention. Typically, mature, primed dendritic cells used to generate the antigen-reactive, polarized T cells are syngeneic to the subject to which they are to be administered (*e.g*., are obtained from the subject). Alternatively, dendritic cells having the same HLA haplotype as the intended recipient subject can be prepared *in vitro* using non-cancerous cells (*e.g.,* normal cells) from an HLA-matched donor. In a specific embodiment, antigen-reactive T cells, including CTL and Th-1 helper cells, are expanded *in vitro* as a source of cells for immunostimulation.

### In vivo Administration of Cell Populations

In another aspect of the invention, methods are provided for administration of mature, primed dendritic cells or activated, for example, polarized T cells, or a cell population containing such cells, to a subject in need of immunostimulation. Such cell populations can include both mature, activated dendritic cell populations and/or activated, for example, polarized T cell populations. In certain embodiments, such methods are performed by obtaining dendritic cell precursors or immature dendritic cells, differentiating and maturing those cells by contact with a tissue culture substrate, a dendritic cell differentiation inducing agent, and predetermined antigen to form a mature activated dendritic cell population primed to induce an antigen specific T cell response. The immature dendritic cells can be contacted with antigen prior to, during, or following activation. Mature or activated dendritic cells can be administered directly to a subject in need of immunostimulation.

In a related embodiment, the activated or mature dendritic cells can be contacted with lymphocytes from a subject to stimulate T cells within the lymphocyte population. The activated, polarized lymphocytes, optionally followed by clonal expansion in cell culture of antigen-reactive CD4⁺ and/or CD8⁺ T cells, can be administered to a subject in need of immunostimulation. In certain embodiments, activated, polarized T cells are autologous to the subject.

In another embodiment, the dendritic cells, T cells, and the recipient subject have the same MHC (HLA) haplotype. Methods of determining the HLA haplotype of a subject are known in the art. In a related embodiment, the dendritic cells and/or T cells are allogenic to the recipient subject. For example, the dendritic cells can be allogenic to the T cells and the recipient, which have the same MHC (HLA) haplotype. The allogenic cells are typically matched for at least one MHC allele (*e.g.,* sharing at least one but not all MHC alleles). In a less typical embodiment, the dendritic cells, T cells and the recipient subject are all allogeneic with respect to each other, but all have at least one common MHC allele in common.

According to one embodiment, the T cells are obtained from the same subject from which the immature dendritic cells were obtained. After maturation and polarization *in vitro,* the autologous T cells are administered to the subject to provoke and/or augment an existing immune response. For example, T cells can be administered, by intravenous infusion, for example, at doses of about 10⁸ to about 10⁹ cells/m² of body surface area (*see, e.g.,* Ridell et al., Science 257:238-241 (1992), incorporated herein by reference). Infusion can be repeated at desired intervals, for example, monthly. Recipients can be monitored during and after T cell infusions for any evidence of adverse effects.

According to another embodiment, dendritic cells matured by contact with a new, unused clean tissue culture substrate according to the present invention can be injected directly into a tumor, or other tissue containing a target antigen. Such partially mature cells can take up antigen and present that antigen to T cells *in vivo.*

### EXAMPLE

The following example is provided merely as illustrative of various aspects of the invention and shall not be construed to limit the invention in any way.

### Example 1: Maturation of Monocytic Dendritic Cell Precursors By Contact With a Tissue Culture Substrate

In this example, monocytic dendritic cell precursors in a cell population enriched for the precursors are differentiated to form immature dendritic cells in the presence of GM-CSF and IL-4. The immature dendritic cells are collected from a tissue culture system, washed, counted and combined with a predetermined antigen in a new, clean tissue culture vessel. The cells are cultured in the presence of a predetermined soluble or particulate antigen under conditions typical for dendritic cell maintenance, typical dendritic cell culture media supplemented with GM-CSF and IL-4. No dendritic cell maturation agent is added to the media. The dendritic cells are determined to have matured without the addition of the dendritic cell maturation agent by determining the presence of cell surface markers characteristic of mature dendritic cells.

Briefly, immature DCs are prepared by contacting peripheral blood monocytes with plastic in the presence of OptiMEM® media (Gibco-BRL) supplemented with 1% human plasma. Unbound monocytes can be removed by washing. The bound monocytes can be cultured in X-VIVO 15® media in the presence of 500 Units of GM-CSF and 500 Units of IL-4 per milliliter for about 6 to 7 days.

The immature DCs are collected by rinsing and the cells are washed with culture media. Cells are counted and the washed DCs are combined with a tumor cell lysate from, for example, a glioblastoma surgically removed from the patient to receive treatment. The DCs and lysate in culture media with GM-CSF and IL-4 as above, and are added to a new clean unused tissue culture dish and cultured for about 12 to about 20 hours. In an alternative embodiment interferon gamma can be added to induce a Th-1 enhanced response.

The activated DCs are collected, washed and prepared for administration to the patient. A sample of the activated DCs is used to test for the phenotype of the cells by labeling with antibody specific for each of CD14, CD83, CD86 and HLA-DR. The labeled cells are analyzed by flow cytometry to determine their phenotype. The activated DCs demonstrate little or no CD14, are positive for CD83, and express high levels of CD86 and HLA-DR as would be expected for mature dendritic cells.

The remaining portion of the activated DCs is administered to the patient at amounts of from 1 x 10⁶ cells to 1 x 10¹⁰ cells per injection. Induction of an antigen specific immune response is evaluated by measuring the T cell responses by MHC tetramer analysis and/or antibody responses to the antigen by ELISA.

### Example 2: Determination of Cell Surface Phenotype of Monocytic Dendritic Cell Precursor Cells Activated Without a Maturation Agent

In this example monocytes were purified through adhesion to a plastic tissue culture vessel, collected and washed, and resuspended in a new tissue culture vessel for an additional culture period. The cell surface expression of the mature dendritic cell marker CD83 was determined.

To generate immature DC, monocytes were purified through adhesion to plastic and cultured for about 7 days in dendritic cell culture medium with GM-CSF and IL-4, and 1% human AB serum. After about 7 days, the cells, which were nearly all floating free in the medium, were harvested and replated in unused, clean polystyrene tissue culture flasks with dendritic cell culture medium with GM-CSF and IL-4, and about 1% human AB serum. Visual observation of the replated cells under an inverted microscope revealed the majority of the cells were tightly adherent to the tissue culture surface. Twenty hours after replating the cells displayed induction of the DC marker CD83. The following table presents CD83 staining on the various samples of isolated monocytes.

| Sample | % CD83⁺ |
|---|---|
| A | 56.7 |
| B | 47.6 |
| C | 41 |
| D | 21 |
| E | 15.4 |
| F | 24.1 |
| G | 40.5 |
| H | 19.6 |
| I | 20.5 |
| J | 19.4 |
| K | 17.9 |
| L | 11.6 |
| M | 12.5 |
| N | 23.6 |
| O | 17.1 |
| P | 13.8 |

### Example 3: Clinical Use of Dendritic Cells Activated Without Maturation Agent

In this example the safety and efficacy of a dendritic cell composition obtained from a glioblastoma multiforme patient activated without a dendritic cell differentiation agent and contacted with a tumor lysate prepared from the patient were tested.

The tumor lysate was prepared from surgically resected tumor tissue. Isolated tumor tissue was minced and placed into a container with a buffer solution containing collagenase are to dissociate the tissue. This mixture was left overnight at room temperature. Following filtering of the ensuring tissue digest, liberated tumor cells were centrifuged into a pellet, The cell pellet was suspended in a small volume of RPMI 1640, and subjected to three freeze-thaw cycles. After freeze-thaw, the tumor lysate was clarified by centrifugation and the protein containing supernatant was filtered through a 0.22 micron filter for sterilization.

A cell population enriched for monocytic dendritic cell precursors was prepared by purification of a leukapheresis product on a FICOLL gradient, followed by adherence to plastic. The adherent cells were monocytic dendritic cell precursors cultured under standard dendritic cell culture conditions for seven days in RPMI 1640 supplemented with 1 0% AB human serum and 500 U/ml each of hGM-CSF and hIL-4.

After seven days the differentiated immature dendritic cells were harvested, washed and either frozen for later use, or mixed with patient tumor lysate and replated in a fresh tissue culture flask in the presence of 1% to 10% human AB serum, and 500 U/ml each of hGM-CSF and IL-4. The final concentration of DC was 0.5 to 2 million cells/ml (typically 1 x 10⁶ cells/ml) and the final concentration of tumor lysate was 10 to 1000 µm/ml (typically 100 µg/ml).

In the first trial described patients received dendritic cells contacted with tumor lysate derived from autologous cultured tumor cells, The study was a dose escalation study where three subjects received 1 x 10⁶ DCs, three subjects received 5 x 10⁶ DCs, and six subjects received 10 x 10⁶ DCs. Immune responses were assessed by measuring delayed-type hyper sensitivity (DTH responses), cytotoxic T cell responses (CTL), and by determining the presence of infiltrating lymphocytes during re-operation in cases of disease recurrence. Each of the assays measure different aspects of the induced immune response. A DTH response is typically evidence of helper T cell (Th) activity, whereas CTL response measures whether the induced response has lead to T cells with the capacity to kill tumor cells. Infiltrating T cells detected in the tumor upon reaction measures whether the activated T cells have the ability to migrate to the tumor site to kill tumor cells *in situ.*

**Table 2. Summary Of Immune Responses - first phase I trial**

| **Patient #** | **DC Dose (# cells/injection)** | **DTH Skin Test Increase** | **Peripheral CTL Activity Increase** | **Intratumoral T cells (at reoperation)^{a}** |
|---|---|---|---|---|
| 1 | 1 × 10⁶ | Positive | Positive | ++++ |
| 2 | 1 × 10⁶ | Negative | Negative | -- |
| 3 | 1 × 10⁶ | Positive | Positive | +++ |
| 4 | 5 × 10⁶ | Negative | Positive | ++ |
| 5 | 5 × 10⁶ | Positive | Positive | n.a. |
| 6 | 5 × 10⁶ | Negative | Positive | n.a. |
| 7 | 10 × 10⁶ | Negative | Negative | -- |
| 8 | 10 × 10⁶ | Negative | Negative | ++ |
| 9 | 10 × 10⁶ | Positive | Positive | +++ |
| 10 | 10 × 10⁶ | Negative | Negative | n.a. |
| 11 | 10 × 10⁶ | Negative | Negative | n.a. |
| 12 | 10 × 10⁶ | Negative | Negative | n.a. |

| | | | | |
|---|---|---|---|---|
| ^{a}scored by semi-subjective assessment of number of infiltrating lymphocytes. n.a., not applicable because no surgery was performed. | | | | |

The second trial was also a dose escalation study, with four subjects receiving 1 x 10⁶ DC per injection, six subjects receiving 5 x 10⁶ DC per injection, and six subjects receiving 10 x 10⁶ DC per injection. In this study, tumor lysate was derived from tumor resected from each particular patient and the lysate was contacted with DC derived from that patient. Immune responses were assessed by tetramer staining for antigen specific CD8⁺ T cells after vaccination and by determining the presence of infiltrating intratumoral T cells at reoperation, if applicable. Typically, reoperation was only carried out after the recurrence of disease. Thirteen of the patients had newly diagnosed glioblastoma multiforme (GBM), two had recurring GBM, and one had a recurring Grade III oligoastrocytoma.

Assessment of the immune response by tetramer staining for antigen-specific T cells allows for the determination of the presence of, and for the quantification of the levels of circulating T cells that express the T cell receptor specific for a given antigen. Such T cells, if also expressing CD8, are presumed to be representative of populations of CTL against these antigens. The antigens selected for this study were known tumor antigens that had been shown by histological methods to be present in the originally resected tumor. Reactivity was tested for tumor associated antigens including Gp100 (melanoma-associated tumor antigen, also found in GBM), Trp-2 (tyroinase-related protein 2), Her-2 (an epidermal growth factor receptor-related receptor tyronine kinase), and CMV (cytomegalovirus) using standard methods.

Tetramer staining for antigen-specific CD8⁺ T cells was attempted in seven patients, and a positive reactivity was detected in five. Of the five reactive patients, two had been immunized with 1 x 10⁶ DC and three had been immunized with 10 x 10⁶ DC. These results indicate that the DC matured by the methods of the present invention were capable of inducing an immune response in a majority of the patients. In most cases that response included an antigen specific cytotoxic T cell response.

The previous examples are provided to illustrate, but not to limit, the scope of the claimed inventions. Other variants of the inventions will be readily apparent to those of ordinary skill in the art and encompassed by the appended claims. All publications, patents, patent applications and other references cited herein and are also incorporated by reference herein in their entirety.

The invention will now be defined by reference to the following clauses:
1. A method for producing a cell population comprising an enriched population of activated dendritic cells comprising: contacting a cell population comprising an enriched population of immature dendritic cells with a tissue culture substrate and a culture medium having a dendritic cell differentiation agent under conditions suitable for adhesion of the immature dendritic cells with the tissue culture substrate and for *in vitro* culture of the dendritic cells for a time period sufficient for maturation of the activated dendritic cells.
2. The method according to clause 1, wherein a predetermined antigen is contacted with the cell population prior to, simultaneous with, or subsequent to contact with the tissue culture substrate.
3. The method according to clause, 2, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide, or an isolated antigen.
4. The method according to clause 3, wherein the cell lysate or membrane preparation is obtained from a brain tumor, a prostate tumor, prostate tissue, an ovarian tumor, a breast tumor, breast tissue, a leukemic cell population, a lung tumor, a melanoma, a bladder tumor, or a tumor cell line.
5. The method according to clause 4, wherein the brain tumor is glioblastoma multiforme or an oligoastrocytoma.
6. The method according to any of clauses 1 through 5, wherein the dendritic cell differentiation agent is GM-CSF, IL-4, IL-13, IL-15 or combinations thereof.
7. The method according to any of clauses 1 through 6, wherein the immature dendritic cells are derived from a cell population enriched for monocytic dendritic cell precursors.
8. The method according to clause 7, wherein the cell population enriched for monocytic dendritic cell precursors is contacted with a dendritic cell differentiation inducing agent.
9. The method according to clause 8, wherein the dendritic cell differentiation inducing agent is GM-CSF, IL-4, IL-13, or IL-15, and combinations thereof.
10. The method according to clause s 7 through 9, wherein the monocytic dendritic cell precursors are from a patient or from an HLA-matched individual.
11. The method according to any of clauses 3 through 9, wherein the tumor cells and the dendritic cells are from a patient.
12. The method according to any of clauses 1 through 11, wherein the immature dendritic cells are further contacted with a directional maturation agent.
13. The method according to clause 12, wherein the directional maturation agent is interferon gamma.
14. A method for activating immature dendritic cells, comprising contacting the immature dendritic cells with a tissue culture substrate and a dendritic cell differentiation inducing agent under conditions suitable for *in vitro* culture of immature dendritic cells and for adhesion of the immature dendritic cells to the tissue culture substrate.
15. The method according to clause 14, further comprising contacting the immature dendritic cells with a predermined antigen prior to, simultaneous with, or subsequent to contacting the immature dendritic cells with the tissue culture substrate.
16. The method according to clause 15, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide, or an isolated antigen.
17. The method according to clause 16, wherein the cell lysate or membrane preparation is derived from tumor tissue or a tumor cell line.
18. The method according to clause 17, wherein the tumor tissue or tumor cell line is obtained from a brain tumor, a prostate tumor, an ovarian tumor, a breast tumor, a lung tumor, a melanoma, a bladder tumor, or a leukemic cell population.
19. The method according to clause 18, wherein the brain tumor is glioblastoma multiforme or an oligoastrocytoma.
20. The method according to any of clauses 14 through 19, wherein the dendritic cell differentiation inducing agent is GM-CSF, IL-4, IL-13, IL-15, or combinations thereof.
21. The method according to any of clause s 14 through 20, wherein the immature dendritic cells are further contacted with a directional maturation agent.
22. The method according to clause 21, wherein the directional maturation agent is interferon gamma.
23. A method for activating T cells, comprising:
   contacting T cells with a dendritic cell population matured by contact with a tissue culture substrate and a dendritic cell differentiation inducing agent under conditions conducive for adhesion of immature dendritic cells to the tissue culture substrate; and
   a predetermined antigen.
24. A method for producing activated antigen specific T cells, comprising:
   contacting isolated immature dendritic cells with a predetermined antigen;
   contacting the isolated immature dendritic cells with a tissue culture substrate and a dendritic cell differentiation inducing agent under conditions conducive for adhesion of the immature dendritic cells to the tissue culture substrate to activate tie immature dendritic cells ; and
   contacting the activated dendritic cells with naive T cells to form activated antigen specific T cells.
25. The method according to clause 24, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen, or an isolated antigen.
26. The method according to clause 25, wherein the cell lysate or membrane preparation is obtained from a brain tumor, a prostate tumor, prostate tissue, an ovarian tumor, a leukemic cell population, a lung tumor, a breast tumor, or a bladder tumor.
27. The method according to clause 26, wherein the brain tumor is glioblastoma multiforme or an oligoastrocytoma.
28. The method according to any of clauses 24 through 27, further comprising as a first step:
   culturing monocytic dendritic cell precursors in the presence of a differentiation inducing agent to form the immature dendritic cells.
29. The method according to clause 28, wherein the differentiation inducing agent is GM-CSF, Interleukin 4, a combination of GM-CSF and Interleukin 4, or Interleukin 13.
30. The method according to clause 28 and 29, wherein the monocytic dendritic cell precursors are isolated from a human subject.
31. The method according to clause 30, wherein the immature dendritic cells and T cells are autologous to each other.

## Claims

1. An *ex vivo* or in vitro method for producing a cell population comprising an enriched population of activated dendritic cells comprising:
contacting a cell population comprising monocytic dendritic cell precursors with a combination of GM-CSF and Interleukin 4, Interleukin 13 or Interleukin 15 to form a cell population comprising an enriched population of immature dendritic cells;
contacting the cell population comprising an enriched population of immature dendritic cells with a new, unused, clean tissue culture substrate and a culture medium having GM-CSF, Interleukin 4, or a combination of GM-CSF and Interleukin 4, Interleukin 13 or Interleukin 15 under conditions suitable for adhesion of the immature dendritic cells with the tissue culture substrate and for *in vitro* culture of the dendritic cells for a time period sufficient for maturation of the activated dendritic cells; and contacting the immature dendritic cells with interferon gamma alone without any dendritic cell maturation agents.

2. The method according to claim 1, wherein the method is without the use of a dendritic cell maturation agent to induce activation and maturation of the dendritic cell population; the new, unused, clean tissue culture substrate is a plastic tissue substrate; conditions suitable for adhesion of the immature dendritic cells with the tissue culture substrate induce activation and maturation of the immature dendritic cells; and further comprising culturing *ex vivo* or *in vitro* the dendritic cells for a time period sufficient for maturation of the immature dendritic cells to form activated dendritic cells.

3. The method according to any one of claims 1 or 2, further comprising contacting the cell population or immature dendritic cells with a predetermined antigen prior to, simultaneous with contacting the cell population or immature dendritic cells with the tissue culture substrate.

4. An *ex vivo* or in vitro method for activating T cells, comprising
i) a method for producing a cell population comprising an enriched population of activated dendritic cells comprising:
(ii) contacting a cell population comprising monocytic dendritic cell precursors with a combination of GM-CSF and Interleukin 4, Interleukin 13 or Interleukin to form a cell population comprising an enriched population of immature dendritic cells;
(iii) contacting the cell population comprising an enriched population of immature dendritic cells with a new, unused, clean tissue culture substrate and a culture medium having a combination of GM-CSF and Interleukin 4, Interleukin 13 or Interleukin 15 under conditions suitable for adhesion of the immature dendritic cells with the tissue culture substrate and for *in vitro* culture of the dendritic cells for a time period sufficient for maturation of the activated dendritic cells;
(iv) contacting the immature dendritic cells with interferon gamma alone without any dendritic cell maturation agents; and
v) contacting T cells with the activated dendritic cells population and a predetermined antigen.

5. The *ex vivo* or in vitro method according to claim 4, wherein the method is without the use of a dendritic cell maturation agent to induce activation and maturation of the dendritic cell population; the new, unused, clean tissue culture substrate is a plastic tissue substrate; conditions suitable for adhesion of the immature dendritic cells with the tissue culture substrate induce activation and maturation of the immature dendritic cells; and further comprising:
(vi) culturing ex *vivo* or *in vitro* the dendritic cells for a time period sufficient for maturation of the activated dendritic cells; and
(vii) contacting T cells with the activated and mature dendritic cells population that present the predetermined antigen.

6. An ex vivo method for producing activated antigen specific T cells, comprising:
i) a method for producing a cell population comprising an enriched population of activated dendritic cells consisting essentially of:
contacting a cell population comprising monocytic dendritic cell precursors with a combination of GM-CSF and Interleukin 4, Interleukin 13 or Interleukin 15 to form a cell population comprising an enriched population of immature dendritic cells;
contacting the cell population comprising an enriched population of immature dendritic cells with a new, unused, clean tissue culture substrate and a culture medium having a combination of GM-CSF and Interleukin 4, Interleukin 13 or Interleukin 15 under conditions suitable for adhesion of the immature dendritic cells with the tissue culture substrate and for *in vitro* culture of the dendritic cells for a time period sufficient for maturation of the activated dendritic cells;
contacting the immature dendritic cells with interferon gamma alone without any dendritic cell maturation agents; and
ii) contacting naive T cells with the activated dendritic cells and a predetermined antigen to form activated antigen specific T cells.

7. The ex *vivo* or in vitro method according to claim 6, wherein the method is without the use of a dendritic cell maturation agent to induce activation and maturation of the dendritic cell population; the new, unused, clean tissue culture substrate is a plastic tissue substrate; conditions suitable for adhesion of the immature dendritic cells with the tissue culture substrate induce activation and maturation of the immature dendritic cells; and further comprising:
contacting the cell population of immature dendritic cells with a predetermined antigen prior to, or simultaneous with contacting the cell population or immature dendritic cells with the tissue culture substrate, wherein the predetermined antigen is a tumor specific antigen, a tumor associated antigen, tumor cells, recombinant cells expressing an antigen, a cell lysate, a membrane preparation, a recombinantly produced antigen, a peptide antigen, or an isolated antigen, preferably wherein the cell lysate or membrane preparation is obtained from a brain tumor, a prostate tumor, prostate tissue, an ovarian tumor, a leukemic cell population, a lung tumor, a breast tumor, or a bladder tumor, preferably wherein the brain tumor is glioblastoma multiforme or an oligoastrocytoma; culturing *ex vivo* or *in vitro* the dendritic cells for a time period sufficient for activation and maturation of the immature dendritic cells to form activated and mature dendritic cells.

8. The method according to any one of claims 3 to 6, wherein the predetermined antigen is a tumor specific antigen, a tumor associated antigen, tumor cells, recombinant cells expressing an antigen, a cell lysate, a membrane preparation, a recombinantly produced antigen, a peptide antigen, or an isolated antigen, preferably wherein the cell lysate or membrane preparation is obtained from a brain tumor, a prostate tumor, prostate tissue, an ovarian tumor, a leukemic cell population, a lung tumor, a breast tumor, or a bladder tumor, preferably wherein the brain tumor is glioblastoma multiforme or an oligoastrocytoma.

9. The method according to any of the preceeding claims, wherein the monocytic dendritic cell precursors have been isolated from a human subject, preferably wherein the immature dendritic cells and T cells are autologous to each other.

10. The method according to claim any of the preceeding claims, wherein the monocytic dendritic cell precursors are from a patient or from an HLA-matched individual.

11. The method according to claim 8, wherein the antigen is tumor cells and wherein the tumor cells and the dendritic cells are from a patient.
